Europäisches Patentamt

European Patent Office

Office européen des brevets

(19)

(11) Publication number: **0 297 068**
**A2**

# EUROPEAN PATENT APPLICATION

(12)

(21) Application number: **88850209.3**

(22) Date of filing: **14.06.88**

(51) Int. Cl.⁴: **C 07 K 7/10**
**A 61 K 37/02**

(30) Priority: **16.06.87 SE 8702520**

(43) Date of publication of application:
**28.12.88 Bulletin 88/52**

(84) Designated Contracting States:
**AT BE CH DE FR GB IT LI NL SE**

(71) Applicant: **KabiGen AB**
**S-112 87 Stockholm (SE)**

**SKANDIGEN AB**
**Norrlandsgatan 15**
**S-106 09 Stockholm (SE)**

(72) Inventor: **Dimaline, Rod Dep. of Physiology**
**University of Liverpool Bronlow Hill P.O.B. 147**
**Liverpool L69 38X (GB)**

**Mutt, Victor**
**Jungfrudansen 18**
**S-171 56 Solna (SE)**

**Jörnvall, Hans**
**Vallstigen 18**
**S-172 46 Sundbyberg (SE)**

**Gafvelin, Guro**
**Karlbergsvägen 72A, 1 tr**
**S-113 35 Stockholm (SE)**

**Andersson, Mats**
**Hammarbyvägen 53**
**S-175 35 Järfälla (SE)**

(74) Representative: **Henningsson, Gunnar et al**
**Bergling & Sundbergh AB Box 7645**
**S-103 94 Stockholm (SE)**

(54) Mammal intestine hormone precursor.

(57) A mammal intestine hormone precursor having the following amino acid sequence structure:
His-Ser-Asp-Ala-Val-Phe-Thr-Asp-Asn-Tyr-Thr-Arg-Leu-Arg-Lys-Gln-X-Ala-Val-Lys-Lys-Tyr-Leu-Asn-Ser-Ile-Leu-Asn-Gly-Y-Z,
wherein X is Met, Thr or Leu and Y and Z are selected from Lys and Arg;
   compositions containing such precursor; and
   a method of generating vasodilation.

Fig.1

EP 0 297 068 A2

## Description

## Mammal intestine hormone precursor.

The present invention relates to a mammal intestine hormone precursor related to the vasoactive intestinal polypeptide (VIP). The invention involves the use of such precursor as a vasodilator and covers also a composition containing same and a method of generating vasodilation.

VIP (vasoactive intestinal polypeptide) was first isolated from porcine upper intestine (1). The hormone consists of 28 amino acid residues and has a C-terminal amidated asparagine residue. Antibodies against VIP have shown that the hormone is widely distributed in the body. High concentrations of VIP have been found in for example the gastrointestinal tract, blood vessels and neural tissues (2). Receptors for VIP have also been observed in several species and several organs (2). These studies have been focusing on the octacosapeptide first isolated. Since VIP is synthesized as a 170 amino acid residues long prohormone (3) (which except of VIP also contains the intestinal hormone PHI/PHM), there is a possibility that other biosynthetical forms of VIP might be detected in assays with antibodies or membranes with receptors that are not highly specific for the octacosapeptide form.

The present invention resides in the discovery of variant forms of vasoactive intestinal polypeptides related to VIP but which are not amidated at their C-terminal extension residue in that they instead have a C-terminal of glycine and another two amino residues selected from Lys and Arg. The variants according to the present invention can be considered as proforms or precursors to the natural occurring vasoactive intestinal polypeptide and they possess high vasodilatory activity.

Thus, according to the present invention there is provided mammal intestine hormone precursors having the following amino acid sequence structure:

His-Ser-Asp-Ala-Val-Phe-Thr-Asp-Asn-Tyr-Thr-Arg-Leu-Arg-Lys-Gln-X-Ala-Val-Lys-Lys-Tyr-Leu-Asn-Ser-Ile-Leu-Asn-Gly-Y-Z.

In this structure X is selected from Met, Thr and Leu, and Y and Z are selected from Lys and Arg.

it is preferred that X is Met and, furthermore, it is preferred that one of Y and Z is Lys and the other one is Arg.

In a particular preferred embodiment of the invention X is Met, Y is Lys and Z is Arg.

The precursor according to this invention is useful as a vasodilator. According to this aspect of the invention a composition for such use comprises an effective therapeutic amount of the secretine precursor of the invention in combination with a non--toxic, pharmaceutical acceptable carrier. In this context the invention also covers a method for generating vasodilation in a patient suffering from a disorder responsive to such treatment. This method comprises administering to said patient an amount of the hormone precursor or a composition thereof sufficient to overcome or combat such disorder.

The present invention thus includes within its scope pharmaceutical compositions, which comprises the mammal intestinal hormone precursor according to this invention in association with a pharmaceutically acceptable carrier. In clinical practice the compositions of the present invention will normally be administered parenterally due to the fact that being a peptide the hormone precursor is sensitive to biologically active environments. Oral or rectal administration may, however, be conveivable using compositions of the slow release type making it possible for the active ingredient to reach the site of primary interest. Among such interesting sites there may be mentioned brain, heart and respiratory tract. The precursor of the invention may also be used for the treatment of impotency and cystic fibrosis and to increase vaginal lubrication.

Preparations according to the invention for the preferred parenteral administration includes sterile aqueous or non-aqueous solutions, suspensions or emulsions. Examples of non-aqueous solvents or suspending media are propylene glycol, vegetable oils, such as olive oil, and injectible organic esters, such as ethyle oleate. These compositions may also contain adjuvants, such as preserving, wetting, emulsifying and dispersing agents. They may be sterilized, for example, by filtering through a bacteria-retaining filter, by incorporation of sterilizing agents in the composition, by irradiation or by heating. They may also be manufactured in the form of sterile solid compositions, which can be dissolved in a sterile injectible medium immediately before use. As well as the more customary intravenious and intramuscular routes the composition may also be administered by intra-ventricular injection.

The perecentages of active ingredient in the compositions of the invention may be varied as long as they constitute a proportion such that a suitable dosage for the desired stimulatory effect on the pancreas is obtained. Obviously several unit dosage forms may be administered at about the same time. Generally, the compositions should contain from about 0.1% to about 80% by weight of active ingredient.

The dose employed depends upon the desired stimulatory effect, the route of administration and the duration of the treatment. The hormone precursor of this invention may be administered each day or, according to the wishes of the medical practitioner, less often e.g. weekly.

The invention will be further illustrated below by specific examples which are not to be construed as limiting with regard to the scope of the invention. In such illustration reference will be made to the drawings, wherein

Fig. 1 shows a chromatogram on CM-chromatography made on active extract fraction;

Fig. 2 shows a chromatogram from a reverse phase HPLC on a specific active fraction from the chromatogram of Fig. 1;

Fig. 3 shows yet another chromatogram from reverse phase column chromatography;

Fig. 4A shows a diagram on receptor assay on normal VIP; and

Fig. 4B shows a diagram of the receptor binding of peptides related to VIP and a non-related peptide.

EXAMPLE 1 (Puricifation methods)

a) Chemicals and reagents.

(3-iodotyrosyl-$^{125}$I)-VIP was obtained from Amersham, United Kingdom

b) CM-chromatographies.

Two CM-cellulose chromatographies were carried out. The first one was performed on a CM-cellulose column (2 x 20 cm) in a 0.02 M phosphate buffer, pH 6.4, and eluted with a salt gradient of 0-0.3 M NaCl. The second CM-cellulose column ( 2.5 x 35 cm) was equilibrated with 0.02 M NH$_4$HCO$_3$ and the fractions were eluted with 0.02 M, 0.04 M and 0.2 M NH$_4$HCO$_3$.

c) High performance liquid chromatography (HPLC).

Reverse phase HPLC was carried out on a LKB Ultropac TSK ODS 120 T column (7.5 x 300 mm, 10 μm particle size) and a Vydac C18 column (4.6 x 250 mm, 5μm particle size) with 0.1% tri-fluoroacetic acid (TFA) in water (Solvent A) and 0.1% TFA in acetonitrile (Solvent B).

EXAMPLE 2 (Analysis methods)

a) Receptor assay.

Cell membranes were prepared from rat liver using the method described by D.M. Neville (4). Protein concentration was determined with the Bradford method (5) with BSA as a standard. The binding studies were performed with 0.16 mg/ml liver plasma membrane proteins, radioactive VIP ($\sim$ 12 pM, final concentration) and the sample at various concentrations in a final volume of 0.5 ml. The assay buffer was a 100 mM Tris buffer, pH 7.5, with 1 mg/ml bacitracin, 100 KIE/ml trasylol and 3% BSA. The incubation was made at 30°C for 30 minutes. Then the membranes with bound VIP were separated from unbound VIP by centrifugation in an Eppendorf microfuge for 5 minutes. The pellets were put into a gammacounter and the radioactivity was determined.

The results from this receptor assay are shown in Figs. 4A and B. The diagram of Fig. 4A relates to the normal VIP-form, in comparison with the extended VIP-variant according to the invention.

b) Amino acid composition analysis.

Hydrolysis was carried out at 110°C for 24 h in evacuated tubes with 6 M HCl containing 0.1% phenol. The hydrolysate was analyzed in a Beckman 121 amino acid analyzer.

c) N-terminal analysis and amino acid sequence analysis.

The peptide was degraded in an Applied Biosystems model 470 A gasphase sequencer and phenyl-thiohydantoin derivatives of the amino acids were analyzed by HPLC on a Nucleosil C18 column with an acetonitrite gradient in sodium acetate (6). N-terminal analysis and partial amino acid sequence analysis was also carried out with the manual dimethylaminoazobenzene isothiocyanate (DABITC) method (7) using byproducts to assist identification (8).

d) Bioassay.

The ability of VIP to increase the secretion of pancreatic juice in the anasthetized cat has been documented (1). This was measured in the secretin bioassay (9) and the sample was compared to a secretin standard and to pure porcine VIP. The results of this bioassay are shown in Table 3.

Example 3 (Purification and analysis procedure

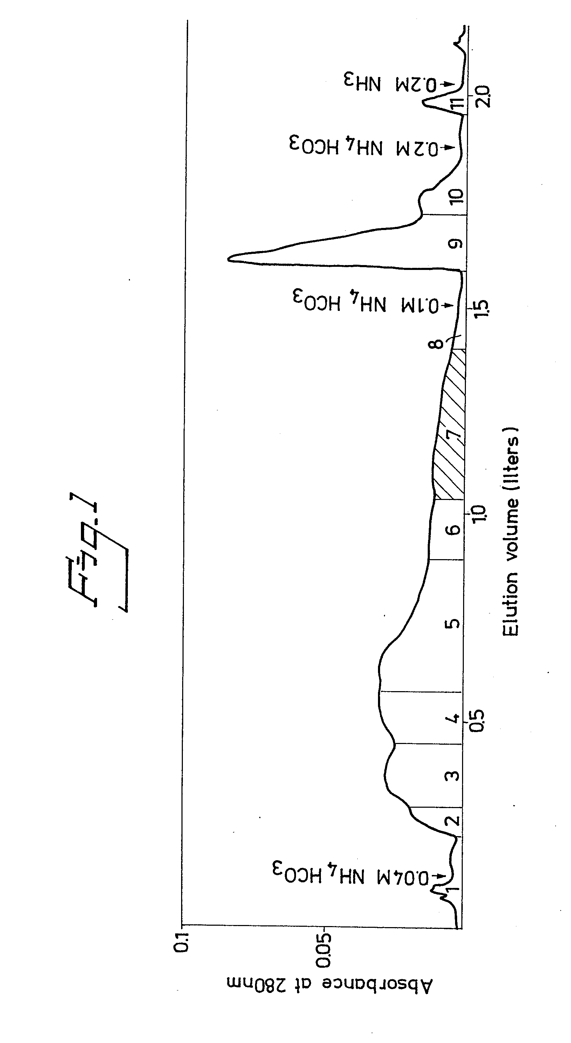

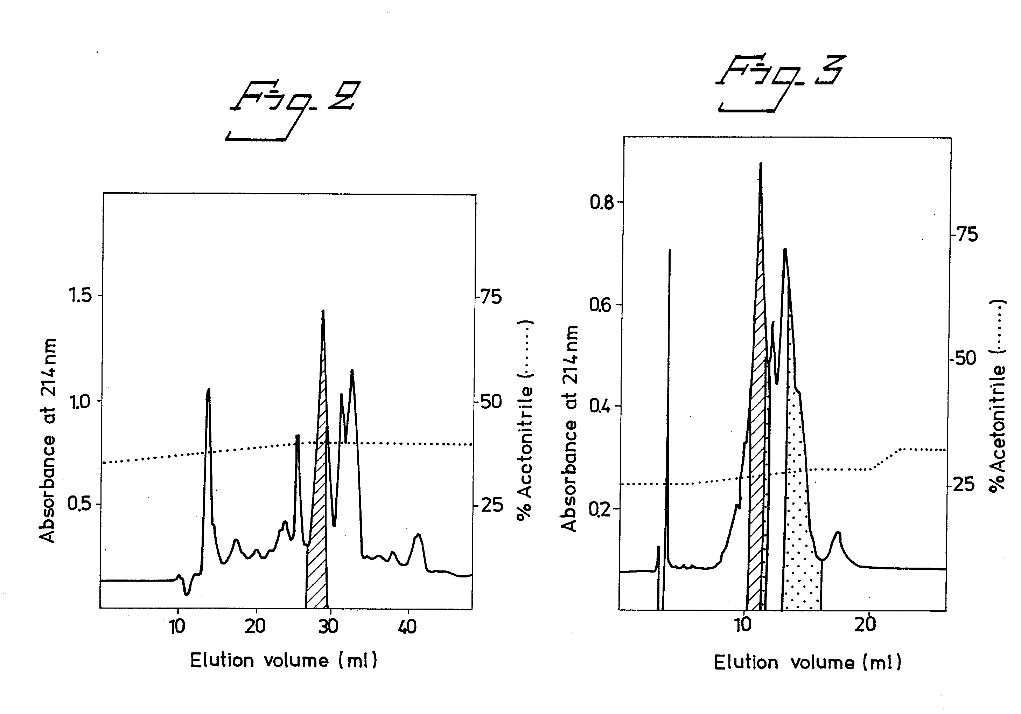

A fraction from the purification of porcine intestine extract, which has earlier been described as the starting material for the purification of VIP (1), was subjected to two CM-chromatographies. In the first chromatography the "true" VIPfraction (Fraction 6) was separated from the other fractions. Fraction 4, which eluted at about 0.15 M NaCl, reacted both in the VIP-receptor assay and a VIP-RIA (10) and was further purified. The chromatogram of Fraction 4 from the second CM-chromatography is shown in Fig. 1. Fractions 6, 7, 8 and 9 displaced labelled VIP in the receptor assay. Fractions 7 and 9 also bound to the VIP-antibodies in the RIA. Fraction 7 was subjected to further purification steps. Fraction 7 was run on a reverse phase HPLC column with 10μm pore size and eluted with a gradient of 35 to 40% Solvent B for 20 min at a flow rate of 1.5 ml/min. The chromatogram is shown in Fig. 2. Each fraction was tested in the VIP receptor assay.

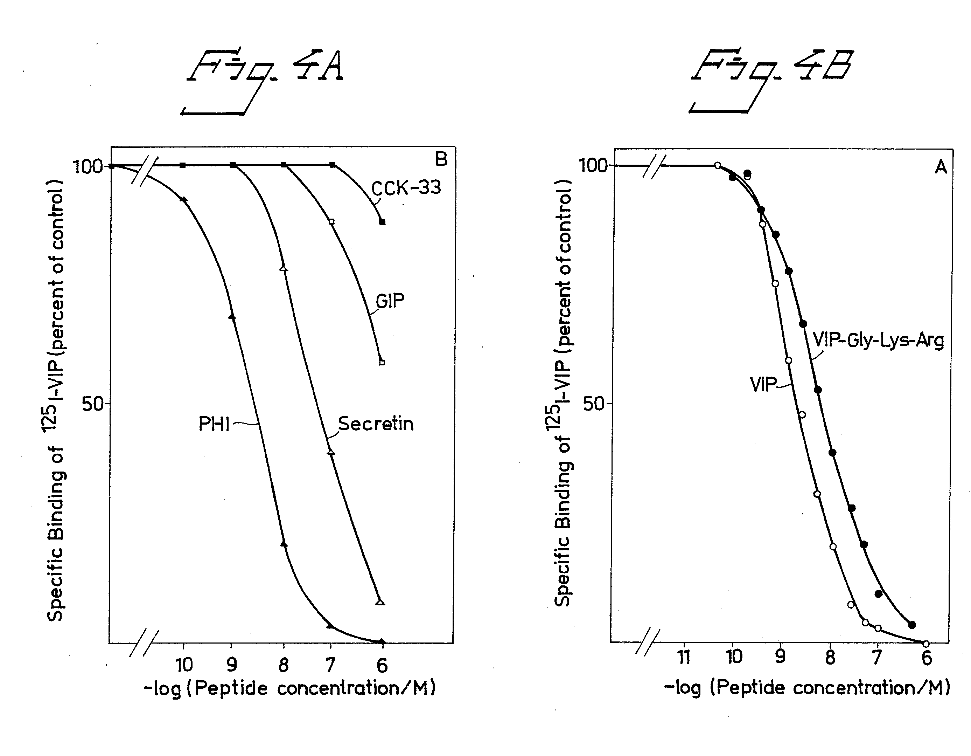

The fraction, indicated by the sectioned area in figure 2, was further purified on another reverse phase colum, 5μm pore size, using a gradient system starting on 25% Solvent B for 5 min, then 25 to 28% Solvent B over 10 min and then 5 min with isocratic conditions before the column was washed with 32% Solvent B. The flow rate was 1 ml/min. When tested in the receptor assay, two main fractions showed receptor binding, as shown in Fig. 3. The fraction indicated by the sectioned area in the figure had the highest content of VIP receptor binding material according to the assay. It was obtained as a pure peptide after a rerun on the same reverse phase column with a gradient of 25 to 30% Solvent B over 20 min. The peptide was then subjected to N-terminal sequence analysis by the manual DABITC method. The six first amino acid residues were identified as the same residues as in VIP, i.e. His-Ser-Asp-Ala-Val-Phe. The total amino acid composition analysis showed some differences compared to the composition of VIP as shown in Table 1. Thus, in contrast to VIP, the purified peptide contains one extra Lys, one extra Arg, at least one Gly (VIP contains no GLy) and it also showed a surprisingly high content of Glu/Gln. The whole amino acid sequence was finally established by analysis in the gas phase amino acid sequencer. As shown in Table 2 the sequence is identical to that of VIP until the C-terminal Asn-amide. Instead of the amide structure, the purified peptide continues with Gly-Lys-Arg, and Arg was the last residue detected.

It is to be noted that the invention is not limited to the specific details and features of the example described above and that modifications can be made to the polypeptide structure without deviating from the inventive idea. Thus, the invention is not limited otherwise than according to the scope of the appended patent claims.

TABLE 1

Aminoacid analysis (Ninhydrin)

| | VIP-variant (nmol) | VIP-var | VIP |
|---|---|---|---|
| Trp | — | | |
| Lys | 4.09 | 3.27 | 3 |
| His | 1.06 | 0.85 | 1 |
| Arg | 3.19 | 2.55 | 2 |
| Asp/Asn | 5.33 | 4.26 | 5 |
| Thr | 2.07 | 1.66 | 2 |
| Ser | 2.14 | 1.71 | 2 |
| Glu/Gln | 2.29 | 1.83 | 1 |
| Gly | 1.89 | 1.51 | — |
| Ala | 2.56 | 2.05 | 2 |
| Val | 2.25 | 1.80 | 2 |
| Met | 1.12 | 0.90 | 1 |
| Ile | 1.39 | 1.11 | 1 |
| Leu | 3.49 | 2.79 | 3 |
| Tyr | 2.18 | 1.74 | 2 |
| Phe | 1.21 | 0.97 | 1 |
| Pro | — | — | — |

TABLE 2

Sequenator results

| Amino acid | pmoles |
|---|---|
| H | 111.0 |
| S | 37.8, 30.1 |
| D | 166.9 |
| A | 210.9 |
| V | 297.3 |
| F | 229.5 |
| T | 57.3 |
| D | 77.2 |
| N | 155.0 |
| Y | 120.7 |
| T | 43.8 |
| R | 4.8 |
| L | 106.7 |
| R | 6.1 |
| K | 300.6 |
| Q | 88.8 |
| M | 86.2 |
| A | 88.2 |
| V | 72.6 |
| K | 130.3 |
| K | 160.0 |
| Y | 56.1 |
| L | 59.8 |
| N | 60.9 |
| S | 13.5, 44.4 |
| I | 39.3 |
| L | 41.0 |
| N | 48.6 |
| G | 33.0 |
| K | 12.2 |
| R | detected |

5

TABLE 3

| Bioassay | Response<br>Micromoles alkali secreted |
|---|---|
| 5 µg VIP | 80 |
| 10 µg VIP | 180 |
| 5 µg VIP GKR | 46 |
| 10 µg VIP GKR | 95 |
| 5 µg VIP | 110 |

(VIP = purified VIP = standard VIP VIP GKR = VIP variant)

## Claims

1. A mammal intestine hormone precursor having the following amino acid sequence structure:
His-Ser-Asp-Ala-Val-Phe-Thr-Asp-Asn-Tyr-Thr-Arg-Leu-Arg-Lys-Gln-X-Ala-Val-Lys-Lys-Tyr-Leu-Asn-Ser-Ile-Leu-Asn-Gly-Y-Z,
wherein X is Met, Thr or Leu and Y and Z are selected from Lys and Arg.

2. The precursor of claim 1, wherein Y is Lys and Z is Arg.

3. The precursor of claim 1, wherein Y is Arg and Z is Lys.

4. The precursor according to any preceding claim, wherein X is Met.

5. The precursor according to any preceding claim for use as a vasodilator.

6. A composition for the generation of vasodilation in mammals including man, comprising an effective therapeutic amount of the hormone precursor of any preceding claim in combination with a non-toxic carrier therefore.

7. A method of generating vasodilation in a patient suffering from a disorder responsive to such treatment, which comprises administering to said patient an amount of the hormone precursor of any of claims 1 to 4 or the composition of claim 6 sufficient to combat such disorder.

Fig.1

Fig. 2

Fig. 3

Fig. 4A

Fig. 4B

0297068